**Europäisches Patentamt**

**European Patent Office**

**Office européen des brevets**

(19)

(11) Veröffentlichungsnummer: **0 009 797**
**B1**

(12) **EUROPÄISCHE PATENTSCHRIFT**

(45) Veröffentlichungstag der Patentschrift:
14.10.81

(51) Int. Cl.³: **C 07 D 323/06,** C 07 D 317/12

(21) Anmeldenummer: 79103732.8

(22) Anmeldetag: 01.10.79

(54) Verfahren zur gleichzeitigen Herstellung von Trioxan und cyclischen Formalen.

(30) Priorität: 05.10.78 DE 2843468

(43) Veröffentlichungstag der Anmeldung:
16.04.80 Patentblatt 80/8

(45) Bekanntmachung des Hinweises auf die Patenterteilung:
14.10.81 Patentblatt 81/41

(84) Benannte Vertragsstaaten:
AT BE CH DE FR GB IT NL SE

(56) Entgegenhaltungen:
AT-B-252 913
DE-A-1 543 664
DE-A-1 668 873
DE-A-1 793 052
DE-A-1 942 387
DE-A-2 001 070
DE-A-2 331 722
DE-B-2 103 687
US-A-2 347 447

(73) Patentinhaber: **HOECHST AKTIENGESELLSCHAFT,**
**Postfach 80 03 20, D-6230 Frankfurt/Main 80 (DE)**

(72) Erfinder: **Mück, Karl-Friedrich, Dr., Wittenberger**
**Strasse 17, D-6200 Wiesbaden (DE)**
Erfinder: **Sextro, Günter, Dr., Erbsenacker 43,**
**D-6200 Wiesbaden (DE)**
Erfinder: **Burg, Karlheinz, Dr., Eichenweg 18,**
**D-6200 Wiesbaden (DE)**

## Verfahren zur gleichzeitigen Herstellung von
## Trioxan und cyclischen Formalen .

Es ist bekannt, Trioxan durch Cyclisierung von Formaldehyd in wäßrig saurer Lösung bei erhöhten Temperaturen herzustellen (vergl. Walker, Formaldehyde Reinhold Publ., New York, 3. Auflage 1964, S. 198/199). Als saure Katalysatoren finden hierbei z. B. starke Mineralsäuren wie Schwefelsäure und Phosphorsäure oder starke organische Säuren wie p-Toluolsulfonsäure oder saure Ionenaustauscher Verwendung (vgl. z. B. US-Patentschrift 23 47 447, DE-AS 11 35 491). Üblicherweise wird das Trioxan durch Destillation aus der Reaktionslösung entfernt. Der Synthesedampf enthält neben Trioxan auch Formaldehyd und Wasser sowie Nebenprodukte der Synthese und wird meist, wie etwa in der britischen Patentschrift 10 12 372 beschrieben, in einer Kolonne mit Verstärker- und Abtriebsteil rektifiziert. Die anfallende trioxanreiche Fraktion wird anschließend durch Extraktion und/oder ein anderes bekanntes Trennverfahren aufgearbeitet.

Weiterhin ist bekannt, cyclische Formale aus Diolen und Formaldehyd bzw. Formaldehyd liefernden Substanzen in Gegenwart saurer Katalysatoren herzustellen (vgl. Walker, Formaldehyde Reinhold Publ. New York 3. Auflage 1964 S. 265, 268). Als Katalysatoren finden die bereits erwähnten sauren Substanzen Verwendung. Um hohe Ausbeuten an den gewünschten Formalen zu erhalten, ist es notwendig, die entsprechenden Alkohole in mindestens stöchiometrischen Mengen oder im Überschuß einzusetzen (vgl. US-Patentschrift 25 66 559 und DE-AS 12 93 143). Die Aufarbeitung der Formale mit niedrigem Siedepunkt und solcher, die wasserdampfflüchtig sind oder mit Wasser Azeotrope bilden, wird gemäß den Verfahrensweisen der US-Patentschrift 23 95 265 oder der DE-AS 11 72 687 durchgeführt. Der Synthesedampf, der das Formal, Wasser, Formaldehyd und Nebenprodukte der Synthese enthält, wird rektifiziert, und das formalreiche Destillat wird durch Extraktion und Destillation weiter gereinigt.

Nachteil der beschriebenen Verfahren ist es, daß beide Substanzklassen getrennt voneinander hergestellt werden und die für die Formale notwendigen Diole in mindestens stöchiometrischen Mengen verwendet werden müssen.

Eine gleichzeitige Synthese von Trioxan und cyclischen Formalen, z. B. Dioxolan, ist von besonderer technischer Bedeutung für die Herstellung von Polyacetalen, da diese Substanzen vorteilhaft als Comonomere bei deren Herstellung eingesetzt werden. Da Trioxan bekanntlich als Ausgangssubstanz zur Herstellung von Formalen verwendet wird (vgl. DE-AS 12 93 143 und DE-AS 12 79 025), war zu erwarten, daß die Trioxansynthese bei gleichzeitiger Formalsynthese erheblich gestört wird.

Überraschenderweise wurde nun gefunden, daß bei einem Verfahren zur gleichzeitigen Synthese von Trioxan und cyclischen Formalen, das dadurch gekennzeichnet ist, daß man ein Gemisch von 30- bis 80prozentiger Formaldehydlösung und 1 bis 25 Gew.-%, vorzugsweise 2−15 Gew.-%, bezogen auf 100prozentigen Formaldehyd, an Diol und/oder Epoxyden mit sauren Katalysatoren bei Temperaturen von 30 bis 150° C, vorzugsweise 100−120° C, umsetzt, vorstehende Nachteile nicht auftreten.

Als Katalysatoren werden die hierfür bekannten Verbindungen wie beispielsweise Phosphorsäure, p-Toluolsulfonsäure oder saure Ionenaustauscher, vorzugsweise Schwefelsäure, in Mengen von 2−30 Gew.-%, vorzugsweise 2−10 Gew.-%, bezogen auf die Reaktionsmischung, eingesetzt. Die verwendete wäßrige Formaldehydlösung ist vorzugsweise 50−70prozentig.

Die erfindungsgemäß eingesetzten Diole sind 1,2-Diole, 1,3-Diole und $\alpha,\omega$-Diole. Ebenso können statt der 1,2-Diole auch die entsprechenden Epoxyde oder Gemische aus beiden eingesetzt werden. Vorzugsweise werden Diole verwendet, deren cyclische Formale Siedepunkte von kleiner als 150° C haben und/oder mit Wasser niedrigsiedende Azeotrope (150° C) bilden oder wasserdampfflüchtig sind. Als geeignet haben sich z. B. Äthylenglykol, Äthylenoxyd, Propylenglykol-1,2, Propylenoxyd, Propylenglykol-1,3, Butandiol-1,2, Butandiol-1,3, Butandiol-1,4 und Buten(3)diol-1,2 erwiesen. Vorzugsweise werden erfindungsgemäß dabei Äthylenglykol, bzw. Äthylenoxyd, Propylenglykol-1,2 und Butandiol-1,4 eingesetzt und besonders bevorzugt Äthylenglykol bzw. Äthylenoxyd.

Die erfindungsgemäß einzusetzenden Diole werden zweckmäßigerweise in Mengen von 1 bis 25 Gew.-%, vorzugsweise 2 bis 15 Gew.-%, bezogen auf 100prozentigen Formaldehyd, verwendet.

Die gleichzeitige Herstellung von Trioxan und cyclischem Formal kann diskontinuierlich oder kontinuierlich, vorzugsweise kontinuierlich erfolgen. Hierzu wird z. B. in einem Rührkessel oder Umlaufverdampfer ein Gemisch aus wäßrigem Formaldehyd, Diol und saurem Katalysator vorgelegt und in dem Maße, wie Reaktionsprodukt aus dem Reaktor entfernt wird, vorzugsweise durch Destillation, ein Gemisch aus Formaldehyd und Diol nachdosiert. Die Verweilzeiten des zugespeisten Gemisches im Reaktor betragen im allgemeinen 2 bis 240 Minuten, vorzugsweise 15 bis 120 Minuten.

Die mit dem erfindungsgemäßen Verfahren erzielten Ergebnisse sind als überraschend anzusehen, da die Ausbeuten an cyclischem Formal etwa bei 90% der Theorie liegen, ohne daß ein Überschuß an Diol eingesetzt wird und da die Umsetzung von Formaldehyd zu Trioxan unbeeinflußt von der Gegenwart des Diols bleibt. So beträgt beispielsweise die Trioxanausbeute pro Durchgang 30% der Theorie bei Verwendung von 65%igem Formaldehyd und liegt damit praktisch gleich hoch als wenn Trioxan isoliert aus gleichkonzentriertem Formaldehyd hergestellt würde. Dies bedeutet eine günstigere Ausnutzung des eingespeisten Formaldehyds und damit eine wirtschaftlichere Verfahrensweise zur Trioxan- und cyclischen Formal-Herstellung als herkömmliche Verfahren.

Weiterhin ist das erfindungsgemäße Verfahren insofern besonders vorteilhaft, als die Mischungen aus Formaldehyd und Diolen wesentlich beständiger sind als gleichkonzentrierte Formaldehyd-Lösungen allein. Hieraus ergibt sich eine verbesserte Handhabbarkeit der Formaldehyd-Lösungen und die Möglichkeit, Formaldehydkonzentrationen einzusetzen, die beim bekannten isolierten Trioxanherstellprozeß nicht realisierbar sind. Schließlich bietet das Verfahren die Möglichkeit, die Comonomeren, die zur Herstellung von Polyacetalen verwendet werden, nämlich Trioxan und z. B. Dioxolan in den gewünschten Mengenverhältnissen im selben Arbeitsschritt und in denselben Aggregaten herzustellen.

Das Reaktionsgemisch, das Trioxan, cyclisches Formal, Wasser, Formaldehyd und Nebenprodukte der Synthese wie beispielsweise Ameisensäure enthält, wird bei Normaldruck, vermindertem Druck oder Überdruck, vorzugsweise bei Normaldruck, destilliert. Die weitere Aufarbeitung erfolgt in der üblichen Weise, z. B. durch Rektifikation, analog dem Verfahren der britischen Patentschrift 10 12 372. Die anfallende trioxan- und formalreiche Fraktion kann dann mittels Extraktion z. B. mit Methylenchlorid und anschließender Neutralisation und Destillation getrennt und gereinigt werden. Auch andere bekannte Trennverfahren können hierfür Einsatz finden, wie beispielsweise in Process Economics Program Report 23 (1967), S. 181, oder in der DE-OS 15 70 335 beschrieben.

Durch die nachfolgenden Beispiele soll das erfindungsgemäße Verfahren näher erläutert werden. Die Prozentangaben verstehen sich jeweils als Gewichtsprozent.

### Beispiele 1 bis 7

In einer kontinuierlich arbeitenden Laborapparatur mit beheiztem Rührkolben und Zudosiervorrichtung werden 400 g einer Mischung bestehend aus 50% Formaldehyd, 40% Wasser und 10% Schwefelsäure vorgelegt und zum Sieden erhitzt. Entsprechend der abdestillierenden Reaktionsmischung wird eine Mischung bestehend aus 65% Formaldehyd mit verschiedenen Mengen Diol, wie aus der nachfolgenden Tabelle ersichtlich, nachdosiert. Die Verweilzeit der Reaktionsmischung im Reaktor betrug 1 Stunde, die Versuchsdauer jeweils 4 Stunden. Die Ausbeute pro Durchgang an Trioxan und Formal sowie die Art des entstandenen Formals läßt sich gleichfalls der Tabelle entnehmen.

In einem Vergleichsversuch wird das Diol weggelassen, d. h. es wurde nur 65%iger Formaldehyd umgesetzt. Die Trioxanausbeute lag hier bei 30%.

3

| Beispiel | Einsatzmischung | Ausbeute pro Durchgang an Trioxan % | Ausbeute pro Durchgang an Formal % | Formal |
|---|---|---|---|---|
| 1 | 65 % $CH_2O$ 1 % Äthylenglykol *) | 29 | 92 | Dioxolan |
| 2 | 65 % $CH_2O$ 7 % Äthylenglykol *) | 30 | 87 | Dioxolan |
| 3 | 65 % $CH_2O$ 15 % Äthylenglykol *) | 29 | 96 | Dioxolan |
| 4 | 65 % $CH_2O$ 25 % Äthylenglykol *) | 29 | 92 | Dioxolan |
| 5 | 65 % $CH_2O$ 5 % Propandiol(1,2) *) | 30 | 98 | 4-Methyldioxolan |
| 6 | 65 % $CH_2O$ 10 % Propandiol(1,2) *) | 30 | 99 | 4-Methyldioxolan |
| 7 | 65 % $CH_2O$ 15 % Propandiol(1,2) *) | 31 | 84 | 4-Methyldioxolan |
| Vergleich | 65 % $CH_2O$ | 30 | — | — |

*) Rest zu 100% = Wasser.

## Patentansprüche

1. Verfahren zur gleichzeitigen Synthese von Trioxan und cyclischen Formalen, dadurch gekennzeichnet, daß ein Gemisch, bestehend aus 30 bis 80prozentigem Formaldehyd und 1 – 25 Gew.-%, bezogen auf 100prozentigen Formaldehyd, an Diol und/oder Epoxyd mit sauren Katalysatoren bei Temperaturen von 30 – 150° C umgesetzt wird.

2. Verfahren gemäß Anspruch 1, dadurch gekennzeichnet, daß das eingesetzte Diol Äthylenglykol, Propandiol-(1,2) oder Butandiol(1,4) ist.

3. Verfahren gemäß Anspruch 1, dadurch gekennzeichnet, daß als Epoxyd Äthylenoxyd oder Propylenoxyd eingesetzt wird.

4. Verfahren gemäß Anspruch 1 bis 3, dadurch gekennzeichnet, daß als Katalysator Schwefelsäure verwendet wird.

## Claims

1. A process for the simultaneous synthesis of trioxane and cyclic formals, which comprises reacting a mixture consisting of a formaldehyde solution, having a concentration of from 30 to 80% by weight, and of from 1 to 25 weight %, referred to 100% formaldehyde, of diol and/or epoxide with acid catalysts at a temperature of from 30 to 150° C.

2. The process as claimed in claim 1, wherein the diol used is ethylene glycol, propanediol-1,2 or butanediol-1,4.

3. The process as claimed in claim 1, which comprises using as epoxide ethylene oxide or propylene oxide.

4. The process as claimed in claims 1 to 3, which comprises using as catalyst sulfuric acid.

**Revendications**

1. Procédé pour préparer en même temps du trioxanne et des formals cycliques, procédé caractérisé en ce qu'on fait réagir un mélange constitué de formaldéhyde d'une concentration comprise entre 30 et 80% et de I à 25% en poids, par rapport au formaldéhyde à 100%, d'un diol et/ou d'un époxyde, en présence de catalyseurs acides, à des températures de 30 à 150° C.

2. Procédé selon la revendication 1, caractérisé en ce que le diol mis en jeu est l'éthylèneglycol, le propane-diol-1,2 ou le butane-diol-1,4.

3. Procédé selon la revendication 1, caractérisé en ce qu'on utilise, comme époxyde, l'oxyde d'éthylène ou l'oxyde de propylène.

4. Procédé selon l'une quelconque des revendications 1à 3, caractérisé en ce qu'on utilise de l'acide sulfurique comme catalyseur.